# EUROPEAN PATENT SPECIFICATION

(11) **EP 2 774 606 B1**
(45) Date of publication and mention of the grant of the patent: **30.01.2019**
(21) Application number: 14155218.2
(22) Date of filing: 14.02.2014
(51) Int. Cl.: A61K 9/00, A61K 9/20, A61K 9/28, A61K 31/55, A61P 9/10

(54) **Pharmaceutical composition comprising ivabradine hydrochloride polymorph IV**
Pharmazeutische Zusammensetzung mit Ivabradin-Hydrochlorid-Polymorph IV
Composition pharmaceutique comprenant le polymorphe IV d'hydrochlorure d'ivabradine

(43) Date of publication of application: 10.09.2014
(73) Proprietor: Synthon B.V., 6545 CM Nijmegen (NL)
(72) Inventor: Bakker-Holmdahl, Lisa, 6545 CM Nijmegen (NL); Murpani, Deepak, 6545 CM Nijmegen (NL)
(74) Representative: Mendivil Gil, Maria Dolores

(56) References cited:
- WO-A1-2013/064307
- WO-A1-2013/102919
- WO-A2-2011/098582
- CN-A- 103 183 639
- MASCIOCCHI N ET AL: "Disclosing the extensive crystal chemistry of Ivabradine hydrochloride, in its pure and solvated phases", POWDER DIFFRACTION SEPTEMBER 2013 INTERNATIONAL CENTRE FOR DIFFRACTION DATA USA, vol. 28, no. 3, September 2013 (2013-09), pages 200-206, XP009178659, DOI: 10.1017/S0885715613000365

## Description

### BACKGROUND OF THE PRESENT INVENTION

Ivabradine, chemically 3-(3-{[((7*S*)-3,4-dimethoxybicyclo[4.2.0]octa-1,3,5-trien-7-yl)methyl]methylamino}propyl)-7,8-dimethoxy-1,3,4,5-tetrahydro-2*H*-3-benzazepin-2-one of formula (I), 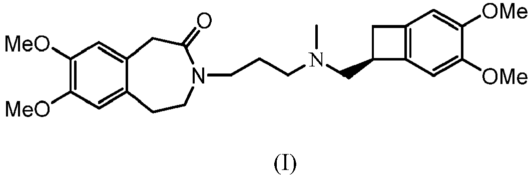 is a pharmaceutically active compound used for the treatment of stable angina pectoris and chronic heart failure. The compound was discovered by Adir and is disclosed in EP534859. The compound may form acid addition salts, for instance Ivabradine hydrochloride, which is the active ingredient in the medicinal product sold under the brand name Corlentor® and Procoralan® by Laboratoires Servier.

Ivabradine hydrochloride exhibits polymorphism. WO2006092493 discloses polymorph β of ivabradine hydrochloride, its process of preparation and compositions comprising this polymorph. Polymorph β, which is a tetrahydrate, is the most stable form and is present in the marketed Corlentor® and Procoralan® tablets. Other polymorphic forms of ivabradine hydrochloride are disclosed in WO2005110993, WO2006092491, WO2006092492, WO2006092494, WO2007042656, WO2007042657 and WO2013064307. WO2011098582 discloses several polymorphic forms of ivabradine hydrochloride. In addition, amorphous ivabradine hydrochloride and the preparation of tablets thereof are described The prior art thus teaches that ivabradine hydrochloride crystallizes very easily in various forms.

WO2013064307 discloses ivabradine hydrochloride polymorph IV, which is a hemihydrate. In addition, the application provides a process to prepare polymorph IV and pharmaceutical compositions comprising this form. WO2013102919 discloses a crystalline form of ivabradine hydrochloride and processes to prepare it. This form, named form II, has a similar X-ray powder diffraction pattern as polymorph IV. It was experienced in our laboratory that polymorph IV, especially in pharmaceutical compositions, transforms over time into the more stable polymorph β. This transformation is even more pronounced in humid environments.

Thus in view of the above, there is a need for pharmaceutical compositions comprising ivabradine hydrochloride polymorph IV, which are stable and suitable for use on a commercial scale.

### BRIEF DESCRIPTION OF THE PRESENT INVENTION

The present invention relates to a tablet composition comprising ivabradine hydrochloride polymorph IV, wherein the X-ray powder diffraction pattern of polymorph IV comprises characteristic peaks at the following 2 theta (± 0.2) angles: 8.8°, 15.6°, 17.1°, 19.9°, 24.2° and 24.5°, measured using a Cu Kα radiation, characterized in that the composition is stabilized by a moisture barrier with a WVTR of less than 0.01 g/m²/day at 38°C/90% RH created by means of packaging the tablet in blister pack material and wherein the tablet is an immediate-release tablet.

Additionally, the invention provides a process for preparing said tablet composition comprising blending ivabradine hydrochloride polymorph IV with one or more pharmaceutically acceptable excipients, followed by compressing the blend into tablets.

Said tablet composition may be used as a medicament, particularly in the treatment of stable angina pectoris and chronic heart failure.

### BRIEF DESCRIPTION OF THE DRAWINGS

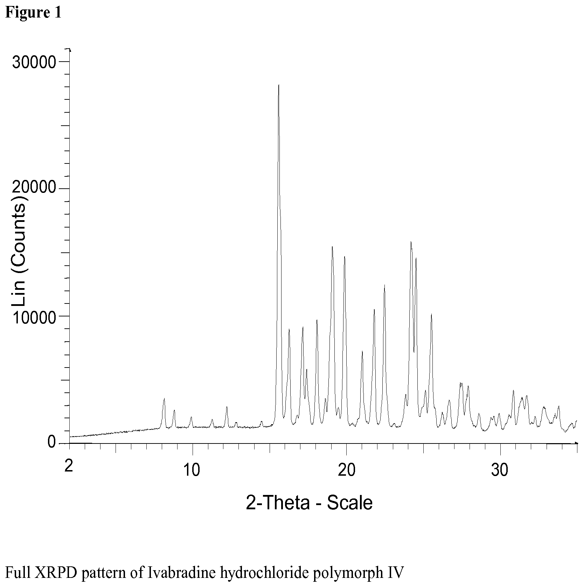

Figure 1 shows the full XRPD pattern of ivabradine hydrochloride polymorph IV. For measurement conditions see the Examples section.

### DETAILED DESCRIPTION OF THE PRESENT INVENTION

The present invention relates to a tablet composition comprising ivabradine hydrochloride polymorph IV, wherein the X-ray powder diffraction pattern of polymorph IV comprises characteristic peaks at the following 2 theta (± 0.2) angles: 8.8°, 15.6°, 17.1°, 19.9°, 24.2° and 24.5°, measured using a Cu Kα radiation, characterized in that the composition is stabilized by a moisture barrier with a WVTR of less than 0.01 g/m²/day at 38°C/90% RH created by means of packaging the tablet in blister pack material and wherein the tablet is an immediate-release tablet.

It was experienced in our laboratory that, especially within the tablet composition, polymorph IV, which is a hemihydrate, transforms over time into the more stable polymorph β, being a tetrahydrate. This transformation is even more pronounced in humid environments. Water vapor transmission rate (WVTR), also moisture vapor transmission rate (MVTR), is a measure of the passage of water vapor through a substance. Various techniques are available to measure WVTR, ranging from gravimetric techniques that measure the gain or loss of moisture by mass, to highly sophisticated instrumental techniques that in some designs can measure extremely low transmission rates. Commercial instruments are available to determine WVTRs using either a pressure-modulated infrared detector or a mechanically modulated infrared detector. Numerous standard methods are described in *e.g.* ISO, ASTM, BS and DIN, like ASTM F1249 and DIN53122. The conditions under which the measurement is made has a considerable influence on the result. Both the temperature and the humidity gradient across the sample need to be measured, controlled and recorded with the result. We have discovered that tablet compositions comprising ivabradine hydrochloride polymorph IV can be stabilized by a moisture barrier with a WVTR of less than 0.35 g/m²/day at 38°C/90% RH. Preferably, the WVTR of the moisture barrier is kept below 0.2 g/m²/day at 38°C/90% RH. More preferably, the WVTR is kept below 0.1 g/m²/day at 38°C/90% RH and most preferably, the WVTR is kept below 0.01 g/m²/day at 38°C/90% RH.

The tablet compositions of the present invention comprise ivabradine hydrochloride polymorph IV and one or more pharmaceutically acceptable excipients. The excipients to be used in accordance with the present invention are well-known and are those excipients which are conventionally used by the person skilled in the art. Depending on the dosage form chosen for the pharmaceutical composition, the person skilled in the art will be able to select suitable pharmaceutically acceptable excipients. Preferably, the dosage form is an immediate-release tablet and the pharmaceutically acceptable excipients are chosen from one or more binders, diluents, disintegrants, glidants, lubricants, stabilizers, surface active agents or pH-adjusting agents.

The present invention further comprises a process to prepare tablet compositions comprising ivabradine hydrochloride polymorph IV and one or more pharmaceutically acceptable excipients. The process comprises blending a composition of ivabradine hydrochloride polymorph IV with one or more pharmaceutically acceptable excipients, followed by compression of the blend into tablets, using equipment and methods well-known in the art.

The tablet compositions of the present invention display dissolution behavior typical for immediate-release formulations. During preparation and storage of the tablet compositions of the present invention, ivabradine hydrochloride remains in polymorphic form IV.

Figure 1 shows the full X-ray powder diffraction pattern of ivabradine hydrochloride polymorph IV. Since the amount of ivabradine hydrochloride in the tablet composition is very small and since excipients are interfering in the analysis, X-ray powder diffraction appeared to be unsuitable as analytical technique to determine polymorphism in the pharmaceutical composition. Instead, Transmission Raman was used, which turned out to be a powerful technique.

In a preferred embodiment of the invention, tablet compositions comprising ivabradine hydrochloride polymorph IV are stabilized by a moisture barrier created by means of packaging the tablet in blister pack material. Blisters belong to the group of primary packaging material in pharmaceutical industry. The WVTR value is a known parameter to express the passage of water vapor through the packaging material in order to control the required quality and shelf life. The type of blister pack material used and its film thickness, determines the WVTR value. Typical WVTR values of a 250 µm PVC blister film and of a duplex 250 µm PVC/90 g/m² PVDC blister film are >3.0 g/m²/day and 0.35 g/m²/day at 38°C/90% RH respectively. These WVTR values are determined according ASTM F1249 ("Water Vapor Transmission Rate through plastic film and sheeting using a modulated infrared sensor"). Cold Form Foil (CFF), also known as Alu-Alu foil, has a WVTR value of 0.005 g/m²/day at 38°C/90%RH. In the present invention, tablet compositions comprising ivabradine hydrochloride polymorph IV are stabilized by a moisture barrier with a WVTR of less than 0.01 g/m²/day at 38°C/90% RH. Preferably, the blister pack material is Cold Form Foil (CFF).

The tablet composition in accordance with the present invention may be used as a medicament. The tablet composition typically may be used in the treatment of stable angina pectoris and chronic heart failure.

The present invention is illustrated by the following Example.

### EXAMPLES

The full XRPD pattern of Ivabradine hydrochloride polymorph IV of Figure 1 was obtained using a Bruker-AXS D8 Vario diffractometer with θ/2θ geometry (reflection mode), equipped with a Vantec PSD detector and applying the following measurement conditions:
Start angle (2θ): 2.0°
End angle (2θ): 35.0°
Scan step width: 0.02°
Scan step time: between 0.2-2.0 seconds
Radiation type: Cu
Radiation wavelengths: 1.5406Å (Kα1), primary monochromator used
Exit slit: 6.0 mm
Focus slit: 0.2 mm
Divergence slit: Variable (V20)
Antiscatter slit: 11.8 mm
Receiving slit: 20.7 mm

### Example 1, preparation of 2.5 mg ivabradine hydrochloride polymorph IV tablets (coated and uncoated) and subsequent stability study in different blister pack materials

### Preparation of ivabradine hydrochloride polymorph IV tablets

51.64 g Ivabradine hydrochloride polymorph IV was mixed with 7.5 g Aerosil 200 VV and 200 g lactose for 10 minutes in a Turbula mixer at 22 rpm. The pre-mix was sieved over a 0.5 mm sieve. 933.33 g Lactose and 300 g maize starch were added and the resulting mixture was mixed for 15 minutes in a Bohle mixer at 25 rpm. The blend was sieved over a Comill 813 sieve and the obtained blend was mixed in a Bohle mixer for 20 minutes at 25 rpm. 7.5 g Magnesium stearate was sieved over a 0.6 mm sieve and was added to the blend. The blend was mixed in a Bohle mixer for 5 minutes at 25 rpm. Tablets were compressed using a Korsch ph 106 with forced feeder, mass 80 mg, 6 mm punch.

The tablets were analyzed by Transmission Raman (Laser: 830 nm; Power at sample: > 600 mW; Fixed Grating: 730 g/mm). The spectra are in accordance with crystalline ivabradine hydrochloride polymorph IV.

The tablets showed a fast dissolution at pH 1.2.

### Coating of ivabradine hydrochloride polymorph IV tablets

24 g Opadry II (not known as moisture protective coating) was dispersed in 136 g water. 800 g of 2.5 mg ivabradine hydrochloride polymorph IV tablets, obtained as described in example 1, were coated with the coating suspension in a Bohle drum coater using a divider disc.

The tablets were analyzed by Transmission Raman (Laser: 830 nm; Power at sample: > 600 mW; Fixed Grating: 730 g/mm). The spectra are in accordance with crystalline ivabradine hydrochloride polymorph IV.

The tablets showed a fast dissolution at pH 1.2.

### Stability study of ivabradine hydrochloride polymorph IV tablets (uncoated and coated) in different blister pack materials

The uncoated and the coated tablets were packed in three different blister pack materials: 250 µm PVC blister film, duplex 250 µm PVC/90 g/m² PVDC blister film and Cold Form Foil, also known as Alu-Alu foil.

The packed tablets were stored at 40°C/75% RH and the samples were analyzed by Transmission Raman (Laser: 830 nm; Power at sample: > 600 mW; Fixed Grating: 730 g/mm).

### Results uncoated tablets

| **Blister pack material** | **Time point** | **Conclusion** |
|---|---|---|
| PVC | 2.5 months at 40°C/75% RH | Mainly ivabradine hydrochloride polymorph β |
| Duplex | 6 months at 40°C/75% RH | Ivabradine hydrochloride polymorph β detected |
| CFF | 6 months at 40°C/75% RH | Ivabradine hydrochloride polymorph IV; no polymorph β detected |

### Results coated tablets

| **Blister pack material** | **Time point** | **Conclusion** |
|---|---|---|
| PVC | 2.5 months at 40°C/75% RH | Mainly ivabradine hydrochloride polymorph β |
| Duplex | 6 months at 40°C/75% RH | Ivabradine hydrochloride polymorph β detected |
| CFF | 6 months at 40°C/75% RH | Ivabradine hydrochloride polymorph IV; no polymorph β detected |

## Claims

1. A tablet composition comprising ivabradine hydrochloride polymorph IV, wherein the X-ray powder diffraction pattern of polymorph IV comprises characteristic peaks at the following 2 theta (± 0.2) angles: 8.8°, 15.6°, 17.1°, 19.9°, 24.2° and 24.5°, measured using a Cu Kα radiation, **characterized in that** the composition is stabilized by a moisture barrier with a WVTR of less than 0.01 g/m²/day at 38°C/90% RH created by means of packaging the tablet in blister pack material and wherein the tablet is an immediate-release tablet.

2. A composition according to claim 1 comprising ivabradine hydrochloride and pharmaceutically acceptable excipients, chosen from one or more binders, diluents, disintegrants, glidants, lubricants, stabilizers, surface active agents or pH-adjusting agents.

3. A composition according to claim 1 or 2, wherein the blister pack material is Cold Form Foil.

4. A process for preparing the tablet composition according to any one of claims 1 to 3 comprising blending ivabradine hydrochloride polymorph IV with one or more pharmaceutically acceptable excipients, followed by compressing the blend into tablets.

5. The composition according to any one of claims 1 to 4, for use as a medicament.

6. The composition according to claim 5 for use in the treatment of stable angina pectoris and chronic heart failure.

## Patentansprüche

1. Tablettenzusammensetzung, umfassend Ivabradin-Hydrochlorid-Polymorph IV, wobei das Röntgen-Pulverbeugungsmuster von Polymorph IV charakteristische Peaks bei den folgenden 2 Theta (± 0,2) -Winkeln umfasst: 8,8º, 15,6º, 17,1º, 19,9º, 24,2º und 24,5º, gemessen unter Verwendung einer Cu Kα-Strahlung, **dadurch gekennzeichnet, dass** die Zusammensetzung durch eine Feuchtigkeitsbarriere mit einer WVTR von weniger als 0,01 g/m²/Tag bei 38 ºC/ 90 % rel. Feuchte, die durch Verpacken der Tablette in Blisterpackungsmaterial erzeugt wird, wobei die Tablette eine Tablette mit sofortiger Freisetzung ist, stabilisiert wird.

2. Zusammensetzung nach Anspruch 1, umfassend Ivabradinhydrochlorid und pharmazeutisch annehmbare Hilfsstoffe, ausgewählt aus einem oder mehreren Bindemitteln, Verdünnungsmitteln, Sprengmitteln, Gleitmitteln, Schmierstoffen, Stabilisatoren, oberflächenaktiven Mitteln oder pH-einstellenden Mitteln.

3. Zusammensetzung nach Anspruch 1 oder 2, wobei das Blisterpackungsmaterial Kaltfolienfolie ist.

4. Verfahren zur Herstellung der Tablettenzusammensetzung nach einem der Ansprüche 1 bis 3, umfassend das Mischen von Ivabradin-Hydrochlorid-Polymorph IV mit einem oder mehreren pharmazeutisch annehmbaren Hilfsstoffen, gefolgt von Komprimieren der Mischung zu Tabletten.

5. Zusammensetzung nach einem der Ansprüche 1 bis 4 zur Verwendung als Medikament.

6. Zusammensetzung nach Anspruch 5 zur Verwendung bei der Behandlung von stabiler Angina pectoris und chronischer Herzinsuffizienz.

## Revendications

1. Composition de comprimé comprenant un polymorphe de chlorhydrate d'ivabradine IV, dans laquelle le diagramme de diffraction de poudre de rayons X du polymorphe IV comprend des pics caractéristiques au niveau des 2 angles thêta (± 0,2) suivants : 8,8º, 15,6º, 17,1º, 19,9º, 24,2º et 24,5º, mesurés à l'aide d'une radiation Cu Kα, **caractérisé en ce que** la composition est stabilisée grâce à une barrière contre l'humidité avec une WVTR inférieure à 0,01 g/m²/jour à 38ºC/90 % de RH créé aux moyens du conditionnement du comprimé dans un matériau d'emballage coque et dans laquelle le comprimé est un comprimé à libération immédiate.

2. Composition selon la revendication 1, comprenant du chlorhydrate d'ivabradine et des excipients pharmaceutiquement acceptables, choisis parmi un ou plusieurs liants, diluants, désintégrants, agents de glissement, lubrifiants, stabilisants, agents tensioactifs ou agents régulateurs du pH.

3. Composition selon la revendication 1 ou 2, dans laquelle le matériau d'emballage coque est un film formé à froid.

4. Procédé pour la préparation de la composition de comprimé selon l'une quelconque des revendications 1 à 3, comprenant le mélange de polymorphe de chlorhydrate d'ivabradine IV avec un ou plusieurs excipients pharmaceutiquement acceptables, suivi par la compression du mélange en comprimés.

5. Composition selon l'une quelconque des revendications 1 à 4, destinée à l'utilisation en tant que médicament.

6. Composition selon la revendication 5, destinée à l'utilisation dans le traitement d'angine de poitrine stable et d'insuffisance cardiaque chronique.
